(19) Europäisches Patentamt

European Patent Office

Office européen des brevets

(11) Numéro de publication: **0 147 331 B1**

(12) **FASCICULE DE BREVET EUROPEEN**

(45) Date de publication du fascicule du brevet:
07.10.87

(51) Int. Cl.⁴: **A 61 K 35/78**, A 61 K 7/48

(21) Numéro de dépôt: **84402725.0**

(22) Date de dépôt: **27.12.84**

(54) Composition cosmétique ou pharmaceutique à base d'extraits végétaux ayant une action sur la fragilité capillaire.

(30) Priorité: **27.12.83 FR 8320826**

(43) Date de publication de la demande:
**03.07.85 Bulletin 85/27**

(45) Mention de la délivrance du brevet:
**07.10.87 Bulletin 87/41**

(84) Etats contractants désignés:
**AT BE CH DE FR GB IT LI NL**

(56) Documents cités:
**FR - A - 2 104 911**
**FR - A - 2 377 201**

**CHEMICAL ABSTRACTS, vol. 81, no. 12, 23 septembre 1974, page 328, no. 68366b, Columbus, Ohio, US**

(73) Titulaire: **L'OREAL, 14, Rue Royale, F-75008 Paris (FR)**

(72) Inventeur: **Aubert, Lucien, 6, Impasse du Verseau, F-06320 Cap d'ail (FR)**
Inventeur: **Anthoine, Philippe, 6, rue Alphonse Karr, F-06000 Nice (FR)**

(74) Mandataire: **Nony, Michel et al, Cabinet Nony 29, rue Cambacérès, F-75008 Paris (FR)**

ACTORUM AG

## Description

La présente demande a pour objet une nouvelle composition, susceptible d'être utilisée dans le domaine cosmétique ou pharmaceutique, contenant des extraits végétaux et ayant une action sur les capillaires en diminuant leur perméabilité et en augmentant leur résistance.

Des extraits de Ruscus aculeatus L associés ou non à un facteur vitaminique ont déjà été proposés en vue du traitement de troubles fonctionnels liés à l'insuffisance veineuse et à la fragilité capillaire.

Par ailleurs, des fractions enrichies en saponines de rhizomes stabilisés de Ruscus aculeatus L ont été décrites comme ayant une activité thérapeutique notamment dans le cas de maladies veineuses, de varices, d'ulcères, d'hémmoroïdes et de différents troubles du système capillaire tels que purpura, épistaxis, engelures ou affections gynécologiques.

Ces compositions de l'art antérieur sont principalement décrites dans le brevet français n° 77.01290 ainsi que dans le B.S.M. n° 3.994.M.

Après différentes études sur les extraits de Ruscus aculeatus L, on a constaté de façon tout à fait surprenante qu'il était possible d'obtenir une remarquable synergie d'action et de complémentarité dans la diminution de la perméabilité des capillaires et dans l'augmentation de leur résistance, en associant les extraits de Ruscus aculeatus L à des extraits de sauge ou Salvia officinalis L.

Il s'est par ailleurs avéré qu'en associant à ces extraits, un extrait de passiflore ou Passiflora incarnata on augmentait de façon plus appréciable l'effet de synergie, l'action étant par ailleurs plus constante dans le temps.

La présente invention a donc pour objet, à titre de produit industriel nouveau, une composition à usage cosmétique ou pharmaceutique, ayant une action sur les capillaires en diminuant leur perméabilité et en augmentant leur résistance, cette composition contenant dans un véhicule approprié pour une application cosmétique ou pharmaceutique, une association d'extraits végétaux constituée:

(i) d'un extrait de fragon (Ruscus aculeatus L), et
(ii) d'un extrait de sauge (Salvia officinalis L).

Selon l'invention, cette association d'extraits végétaux est présente dans la composition aux concentrations suivantes, exprimées en matière sèche:

| | | | |
|---|---|---|---|
| — extrait sec de fragon | | | |
| (Ruscus aculeatus L) | 0,1 | à 3 | % |
| et de préférence | 0,3 | à 2,5 | % |
| — extrait sec de sauge | | | |
| (Salvia officinalis L) | 0,01 | à 5 | % |
| et de préférence | 0,3 | à 3,5 | %. |

Selon une forme particulière de réalisation, la composition contient en outre un extrait mou de passiflore (Passiflora incarnata) à une concentration (exprimée en matière solide) comprise entre 0,1 et 2% et de préférence entre 0,2 et 1,5%.

L'effet vasoprotecteur de ces associations ainsi que leur synergie d'action ont été mis en évidence à l'aide de la méthode des pétéchies dont il sera fait référence ci-après.

L'extrait sec de fragon ou Ruscus aculeatus L, utilisé dans les compositions selon l'invention, est obtenu à partir des rhizomes préalablement broyés et extraits à l'aide d'une solution hydroalcoolique d'un alcool ayant de 3 à 6 atomes de carbone, de préférence à l'aide de n-butanol saturé d'eau.

On peut mentionner comme procédé d'extraction creux décrits dans les brevets français n° 1.377.453, 69.23.340 et 71.29.817, ce dernier brevet ayant tout particulièrement pour objet une purification des extraits obtenus en vue de les enrichir en saponines.

Les extraits de Ruscus aculeatus L obtenus par ces procédés se présentent sous forme d'une poudre beige soluble à 2% dans l'eau et dans l'alcool à 60°. Ces extraits sont essentiellement caractérisés par une teneur en saponines supérieure à 65% et de préférence comprise entre 70 et 80%.

L'extrait sec de sauge au Salvia officinalis L est un extrait obtenu à partir des feuilles et sommités fleuries séchées. L'extraction peut être réalisée à chaud à l'aide d'eau, les jus d'extraction sont alors filtrés, concentrés sous vide puis séchés par atomisation.

On peut également utiliser selon l'invention des extraits hydroalcooliques, des teintures à 60 ou 40% en alcool, des extraits fluides alcooliques (30%) ou propylène-glycoliques (40%).

L'extrait sec de sauge est essentiellement caractérisé par la présence de l'acide ursolique, de flavonoïdes (glucosides de la lutéoline et de l'apigénine), de l'acide rosmarinique, de la picrosalvine ainsi que de divers produits terpéniques tels que la tujone, le bornéol, le salvène, le pinène, l'acétate de bornyle et de linolyle.

L'extrait sec se présente sous forme d'une poudre fine, de couleur brun jaunâtre à marron, soluble jusqu'à 1% dans l'eau et faiblement soluble dans l'alcool à 60° et peu soluble dans l'alcool à 95°.

L'extrait mou de passiflore ou Passiflora incarnata est obtenu par extraction aqueuse ou hydroalcoolique des parties aériennes de la plante, puis concentration en vue d'aboutir à une masse pâteuse ayant une teneur en solides supérieure à 60% et de préférence d'environ 80%.

L'extrait qui se présente sous forme d'une pâte brune très foncée contient en tant que principes actifs de la vitexine, de l'isovitexine, de l'orientine et de l'isoorientine.

Une solution à 2% dans l'éthanol à 50% (en volume) est limpide ou légèrement opalescente.

Lorsque les compositions sont destinées à une application cosmétique, celles-ci se présentent de préférence sous forme d'une émulsion, d'une crème, d'un lait, d'un gel, d'une lotion, d'un cataplasme ou d'une mousse aérosol.

Ces compositions applituées sur la peau ont une action amincissante et anticellulitique notamment lorsqu'elles sont associées à d'autres extraits végétaux et/ou à d'autres principes actifs comme à des composés organiques hydrosolubles dérivés du monométhyltrisilanol tel que par exemple le manuronate de monométhyltrisilanol vendu par la Société EXYMOL sous la dénomination d'«Algisium» (solution aqueuse contenant 1% de manuronate de monométhyltrisilanol) ou le lactate vendu par la même Société sous la dénomination de «Lasilium» (solution aqueuse contanant 1 % de lactate de monométhyltrisilanol).

Ces derniers composés peuvent être présents à une concentration comprise entre 2 et 20 % des solutions à 1 % soit entre 0,02 et 0,2 % en poids exprimé en matière active.

Ces compositions peuvent en outre contenir d'autres ingrédients conventionnels tels que par exemple des parfums, des colorants, des agents conservateurs, des épaississants, des solvants, etc.

Selon un mode de réalisation préféré, les compositions sont destinées à un usage pharmaceutique dans les cas d'insuffisance veineuse ou capillaire.

En particulier, ces compositions trouvent une application en phlébologie, notamment dans les syndromes d'origine veineuse tels que ceux connus sous l'expression de «Jambes Lourdes», les ulcères de jambes, les phlébites, les engelures, en gynécologie dans le cas de certaines dysménorrhées, et en proctologie dans le traitement des hémorroïdes simples et des anites hémorroïdaires.

Les compositions pharmaceutiques destinées a une application systémique peuvent être préparées, par exemple, en ajoutant les extraits tels que définis ci-dessus en tant que substance active, à des supports internes non toxiques, solides ou liquides, généralement utilisés dans ce type de compositions.

Ces compositions peuvent être administrées par voie entérale, parentérale ou topique. Pour une administration par voie entérale, les compositions se présentent sous forme de comprimés, de granulés, de gélules, de dragées, de sirops, de suspensions, de solutions ou de suppositoires.

La posologie est bien entendu fonction de la voie d'administration et de l'activité recherchée.

En proctologie par exemple, les suppositoires peuvent contenir, par unité, dans un excipient constitué de glycérides semi-synthétiques de:

— 0,01 à 0,05 g d'extrait sec de sauge,
— 0,01 à 0,03 g d'extrait sec de fragon,
et éventuellement de:
— 0,01 à 0,03 g d'extrait mou de passiflore.

Les compositions pharmaceutiques peuvent contenir des additifs inertes ou éventuellement pharmaco-dynamiquement actifs. Les comprimés ou granulés peuvent contenir par exemple des liants, des charges, des supports ou des diluants. Les compositions liquides peuvent être présentes par exemple sous la forme d'une solution stérile et miscible à l'eau. En plus des extraits, les gélules peuvent contenir une charge ou un épaississant. Les compositions pharmaceutiques par voie orale peuvent également contenir des agents d'amélioration de la saveur et des substances habituellement utilisées comme conservateurs, stabilisants, régulateurs et émulsionnants.

Les supports et diluants tels qu'énumérés ci-dessus peuvent être constitués de substances organiques ou minérales, par exemple par de la gélatine, du lactose, de l'amidon, du stéarate de magnésium, du talc, de la gomme arabique ou des polyalkylèneglycols. Lorsque les compositions pharmaceutiques sont destinées à une application topique, celles-ci se presentent sous forme d'onguents, de pommades, de teintures, de crèmes, de solutions, de lotions, de sprays ou de suspensions.

Les onguents ou les pommades sont préférés et sont préparés en mélangeant les extraits selon l'invention en tant que constituants actifs à des supports inertes non toxiques appropriés pour un traitement par voie topique.

A titre d'exemple, une crème pour le traitement des jambes lourdes, périphlébites, hypodermites ou engelures, contient dans un excipient approprié pour 100 g,

— 0,3 à 3,5 g d'un extrait sec de sauge, et
— 0,3 à 2,5 g d'un extrait sec de fragon,
et éventuellement de:
— 0,2 à 1,5 g d'un extrait mou de passiflore.

### Mesure de l'activité sur la fragilité capillaire par la méthode des pétéchies

Cette méthode couramment utilisée pour la détermination de la fragilité capillaire a été décrite dans l'article de J.L. PARROT et P. CANU, «Les Facteurs qui élèvent la résistance des capillaires» Arch. Int. Pharmacodyn. n° 1, p. 152 (1964).

On en rappellera cependant ci-après le principe et la méthode.

Le principe consiste, à l'aide d'une chambre à vide, à provoquer l'apparition de pétéchies sur une partie de la peau dorsale de rats ce qui permet la mesure de la résistance capillaire au temps = 0. On applique alors, sur une zone adjacente et délimitée de la peau, la composition à tester ce qui entraîne une variation de cette résistance, variation qui est enregistrée sur différentes parties de la peau traitée à des intervalles de temps réguliers (30 mn, 1h, 1h30, 2h, et 2h30).

L'expérimentation est terminée par une dernière mesure sur une partie de la peau non traitée en vue de vérifier que la mesure témoin n'a pas varié.

L'appareillage permettant la mesure est dérivé de celui décrit par R. CHARLIER, A. HOOSLET et M. COLOT, «Investigations expérimentales sur la fragilité vasculaire» Arch. Int. de Physiologie et de Biochimie, 71, (1), 1963, il comprend une pompe à vide reliée à un réservoir à vide lui même relié à un manomètre qui permet de mesurer la dépression exprimée en mm de Hg. Un deuxième flacon est intercalé entre le réservoir à vide et le manomètre et sert de tampon. Une cellule reliée à un tube de verre commandé par un robinet permet l'application de la pression à la peau. La cellule présente un diamètre d'environ 5 mm et comporte des bords plats de façon à éviter une déformation de la peau.

Pour chaque composition à tester, les mesures de la résistance capillaire ont été réalisées sur 16 rats blancs WISTAR de sexe mâle (poids 360-400 g) dont la partie dorsale inférieure a été tondue et épilée, les animaux avant l'expérimentation étant laissés 48h au repos.

Au début de l'expérimentation, on mesure le seuil de la résistance capillaire pour chaque rat en appliquant une dépression de 350 mm de Hg (1 mm Hg = 133,3223874 Pa) pendant 15 secondes puis en augmentant cette dépression de 5 en 5 mm de Hg jusqu'à l'apparition des pétéchies (4 à 5 pétéchies).

Cette mesure étant effectuée, on applique alors la composition à tester ( 2 mg/cm$^2$) sur une partie adjacente et délimitée de la peau et on mesure toutes les 30 min, à des emplacements différents, la dépression nécessaire pour faire apparaître les pétéchies. L'expérimentation est arrêtée après la 6ème mesure, c'est à dire après 2h30.

On vérifie alors, sur une partie non traitée, que la résistance capillaire mesurée au temps t = 0 n'a pas subi de variation sensible.

Selon cette méthode, on a déterminé la résistance capillaire à l'aide des compositions suivantes:

1) Placébo = excipient ayant la composition suivante:

— Acide polyacrylique (Carbopol® 941)     0,2 g
— Triéthanolamine à 99%     0,6 g
— Propylène glycol     5 g
— Parahydroxybenzoate de méthyle     0,1 g
— Parahydroxybenzoate de propyle     0,2 g
— Acide stéarique     2 g
— Stéarate de glycérol autoémulsionnable     4 g
— Alcool cétylique     1 g
— Huile de Vaseline®     20 g
— Eau déminéralisée stérile Q.S.P.     100 g

2) Crème P = Excipient + 0,5% d'extrait mou de passiflore

3) Crème S = Excipient + 0,5% d'extrait sec de sauge

4) Crème F = Excipient + 1 % d'extrait sec de fragon

5) Crème FP = Excipient + 1 % d'extrait sec de fragon + 0,5% d'extrait mou de passiflore

6) Crème PS = Excipient + 0,5% d'extrait mou de passiflore + 0,5% d'extrait sec de sauge

7) Crème FS = Excipient + 1 % d'extrait sec de fragon + 0,5% d'extrait sec de sauge

8) Crème FSP = Excipient + 1 % d'extrait sec de fragon + 0,5% d'extrait sec de sauge + 0,5% d'extrait mou de passiflore Les résultats observés sont rassemblés dans le tableau A suivant. Les valeurs obtenues (mm/Hg) correspondent à une moyenne des mesures relevées sur les 16 rats traités pour chacune des crèmes.

### TABLEAU A

| temps | | t = 0 (témoin) | t = 30' | t = 1h | t = 1h30 | t = 2h | t = 2h30 | t = > 2h30 (témoin) |
|---|---|---|---|---|---|---|---|---|
| Placébo | 1* | 373,8 | 371,1 | 373,8 | 374,3 | 373,5 | 374,6 | 373,5 |
| | 2** | —— | – 0,7 | 0 | +0,4 | – 0,1 | +0,2 | – 0,1 |
| Crème P | 1 | 379,1 | 382,2 | 387,5 | 390,0 | 390,3 | 385,3 | 380,3 |
| | 2 | —— | +0,8 | +2,2 | +2,9 | +3,0 | +1,8 | +0,3 |
| Crème S | 1 | 364,4 | 366,5 | 370,6 | 374,7 | 376,2 | 376,6 | 366,8 |
| | 2 | —— | +0,6 | +1,7 | +2,8 | +3,3 | +3,3 | +0,6 |
| Crème F | 1 | 376,5 | 380,9 | 388,2 | 392,3 | 394,4 | 391,2 | 378,5 |
| | 2 | —— | +1,1 | +3,1 | +4,2 | +4,8 | +3,9 | +0,5 |
| Crème FP | 1 | 367,8 | 375 | 388,7 | 394,4 | 403,7 | 405,3 | 374,4 |
| | 2 | —— | +1,9 | +5,7 | +7,2 | +9,8 | +10,2 | +1,8 |
| Crème PS | 1 | 370 | 372,8 | 380,6 | 387,8 | 390,6 | 392,8 | 373,4 |
| | 2 | —— | +0,8 | +2,9 | +4,8 | +5,6 | +6,2 | +0,9 |
| Crème FS | 1 | 378,1 | 399,8 | 417,2 | 432,3 | 442,8 | 454,1 | 381,9 |
| | 2 | —— | +5,7 | +10,3 | +14,3 | +17,1 | +20,1 | +1 |
| Crème FSP | 1 | 390,9 | 397,8 | 424,4 | 455 | 480,9 | 481,9 | 403,4 |
| | 2 | —— | +1,8 | +8,5 | +16,4 | +23 | +23,3 | +3,2 |

*1 = Moyenne des valeurs en mm de Hg.
**2 = % d'évolution par rapport au témoin (t = 0).

## Analyse des résultats

1) Le Placébo n'a aucune incidence sur la résistance capillaire.

2) L'extrait mou de passiflore n'augmente que très faiblement la résistance capillaire (maximum 3% environ obtenu après 2h puis chute rapide).

3) L'extrait sec de sauge présente un maximum similaire à l'extrait de passiflore mais reste constant après 2h.

4) L'extrait sec de fragon a une action sensible sur la résistance capillaire (environ 4,5% entre 1h30 et 2h après l'application).

5) L'association de l'extrait de fragon et de l'extrait de passiflore exerce une action sur la résistance capillaire, celle-ci passant à environ 10% après 2h-2h30 après l'application.

6) L'association de l'extrait mou de passiflore et de l'extrait sec de sauge montre également un effet sur la résistance capillaire mais est moins accentué (6% seulement après 2h30).

7) L'association de l'extrait sec de fragon et de l'extrait sec de sauge a un effet de synergie important sur la résistance capillaire par rapport à l'extrait sec de fragon d'une part et à l'extrait sec de sauge d'autre part (augmentation de la résistance capillaire d'environ 20%).

8) L'association de l'extrait sec de fragon, de l'extrait sec de sauge et de l'extrait mou de passiflore induit une très forte augmentation de la résistance capillaire.

Si, aux moyennes relevées pour chacune des crèmes, on applique la méthode de comparaison multiple des moyennes de NEWMAN et KEULS (analyse de variance) on obtient les classements donnés ci-après).

Pour un temps donné, les moyennes relevées pour chaque crème sont classées par ordre croissant.

Les moyennes soulignées par un même trait continu ne sont pas significativement différentes entre elles, dans le cas contraire, la différence est significative avec un risque de 5%.

### 1) Temps 1h

| Crème | S | PS | P | FP | F | FS | FSP |
|---|---|---|---|---|---|---|---|
| Moyenne | 370,6 | 380,6 | 387,5 | 388,8 | 388,2 | 423,4 | 424,4 |

### 2) Temps 1h30

| Crème | S | PS | P | F | FP | FS | FSP |
|---|---|---|---|---|---|---|---|
| Moyenne | 374,7 | 387,8 | 390,3 | 392,3 | 394,4 | 432,3 | 455,0 |

### 3) Temps 2h

| Crème | S | P | PS | F | FP | FS | FSP |
|---|---|---|---|---|---|---|---|
| Moyenne | 376,2 | 390,3 | 390,6 | 394,4 | 403,7 | 442,8 | 480,9 |

### 4) Temps 2h30

| Crème | S | P | F | PS | FP | FS | FSP |
|---|---|---|---|---|---|---|---|
| Moyenne | 376,6 | 385,3 | 391,2 | 392,8 | 405,3 | 454,1 | 481,9 |

Il résulte de ces classements que la crème FS selon l'invention est après 1h30 significativement inférieure à la crème FSP mais est significativement supérieure aux autres crèmes après 2h.

Après 2h30, les crèmes FS et FSP ne sont pas significativement différentes entre elles mais sont significativement supérieures aux autres crèmes.

On va maintenant donner à titre d'illustration et sans aucun caractère limitatif plusieurs exemples de compositions à base des extraits végétaux selon l'invention.

### Exemple 1
#### Crème amincissante

| | |
|---|---|
| — Palmitate d'isopropyle | 3,0 |
| — Huile de soja | 6,0 |
| — Acide stéarique triple pression | 4,0 |
| — Alcool cétylique | 4,0 |
| — Monostéarate de glycérol | 4,0 |
| — Parahydroxybenzoate de méthyle | 0,2 |
| — Parahydroxybenzoate de propyle | 0,1 |
| — Triéthanolamine à 99% | 0,8 |

— *Extraits végétaux:*

| | |
|---|---|
| Extrait sec de sauge (extrait aqueux) | 2,0 |
| Extrait sec de fragon (extrait n-butanolique saturé d'eau) | 2,0 |
| — Parfum | 0,3 |
| — Eau déminéralisée stérile Q.S.P. 100% en poids | |

### Exemple 2
#### Lait corporel relaxant pour «jambes lourdes»

| | |
|---|---|
| — Huile de vaseline | 16,0 |
| — Palmitate d'isopropyle | 2,0 |
| — Lanoline liquide | 1,0 |
| — Acide stéarique triple pression | 2,5 |
| — Stéarate de glycérol | 2,5 |
| — Triéthanolamine à 99% | 0,8 |
| — Parahydroxybenzoate de méthyle | 0,2 |
| — Parahydroxybenzoate de propyle | 0,1 |

— *Extraits végétaux:*

| | |
|---|---|
| Extrait sec de fragon (extrait n-butanolique saturé d'eau) | 0,5 |
| Extrait sec de sauge (extrait aqueux) | 0,5 |

— Parfum    0,5
— Eau déminéralisée Q.S.P.    100% en poids

*Exemple 3*
Gel défatigant pour «jambes lourdes»

— Acide polyacrylique (Carbopol 940)    1,0
— Triéthanolamine à 99%    1,0
— Propylène glycol    8,0
— Parahydroxybenzoate de méthyle    0,1
— Parahydroxybenzoate de propyle    0,2
— Parfum    0,3

— *Extraits végétaux:*

    Extrait sec de fragon (extrait n-butanolique saturé d'eau)    1,5
    Extrait de sauge (extrait aqueux)    0,3
    Extrait mou hydroalcoolique de passiflore (contenant 76,5% de matière solide)    1,5
— Eau déminéralisée stérile Q.S.P. 100% en poids

*Exemple 4*
Crème pour la couperose

— Acide stéarique triple pression    3,0
— Alcool cétylique    3,0
— Stéarate de glycérol    3,0
— Mono-oléate de sorbitan polyoxyéthyléné    3,0
— Huile de tournesol    10,0
— Propylène glycol    4,0
— Sorbitol    3,0
— Parahydroxybenzoate de méthyle    0,2
— Parahydroxybenzoate de propyle    0,1
— Parfum    0,3

— *Extraits végétaux:*

    Extrait sec de fragon (extrait n-butanolique saturé d'eau)    0,3
    Extrait sec de sauge (extrait aqueux)    0,1
    Extrait mou hydroalcoolique de passiflore (contenant 76,5% de matière solide)    0,1
— Eau déminéralisée stérile Q.S.P. 100% en poids

*Exemple 5*
Onguent pour varices

— Myristate d'isopropyle    90,5 g
— Silice (vendue sous la dénomination d'AEROSIL 200 par la Sté DEGUSSA)    8,0 g

— *Extraits végétaux:*

    Extrait sec de fragon (extrait n-butanolique saturé d'eau)    0,5 g
    Extrait sec de sauge (extrait aqueux)    1,0 g

*Exemple 6*
Suppositoires pour hémorroïdes
(composition par unité)

— *Extraits végétaux:*

    Extrait sec de fragon (extrait n-butanolique saturé d'eau)    0,02 g
    Extrait sec de sauge (extrait aqueux)    0,02 g
    Extrait mou hydroalcoolique de passiflore (contenant 76,5% de matière solide)    0,02 g

— Triglycérides des acides caprylique et caprique    0,2 g
— Glycérides semi-synthétiques Q.S.P.    2,0 g

**Revendications pour les Etats contractants:**
BE, CH, DE, GB, IL, LI, NL

1. Composition à usage cosmétique ou pharmaceutique ayant une action sur la fragilité capillaire, caractérisée par le fait qu'elle contient dans un véhicule approprié pour une application cosmétique ou pharmaceutique, une association d'extraits végétaux constituée:

(i)  d'un extrait de fragon (<u>Ruscus aculeatus L</u>), et
(ii)  d'un extrait de sauge (<u>Salvia officinalis L</u>).

2. Composition selon la revendication 1, caractérisée par le fait qu'elle contient de 0,1 à 3% et de préférence de 0,3 à 2,5% en poids d'un extrait sec de fragon par rapport au poids total de la composition.

3. Composition selon la revendication 1, caractérisée par le fait qu'elle contient de 0,01 à 5% et de préférence de 0,3 à 3,5% en poids de l'extrait sec de sauge par rapport au poids total de la composition.

4. Composition selon l'une quelconque des revendications 1 à 3, caractérisée par le fait qu'elle contient en outre un extrait mou de passiflore (<u>Passiflora incarnata</u>) à une concentration (exprimée en matière solide) comprise entre 0,1 et 2% et de préférence entre 0,2 et 1,5% en poids par rapport au poids total de la composition.

5. Composition selon l'une quelconque des revendications 1 et 2, caractérisée par le fait que l'extrait de fragon est un extrait résultant de l'extraction à l'aide d'une solution hydroalcoolique d'un alcool ayant de 3 à 6 atomes de carbone et de préférence à l'aide de n-butanol saturé d'eau.

6. Composition selon la revendication 5, caractérisée par le fait que l'extrait sec de fragon a une teneur en saponines supérieure à 65% et de préférence comprise entre 70 et 80% en poids par rapport à l'extrait sec.

7. Composition selon l'une quelconque des revendications 1 et 3, caractérisée par le fait que l'extrait de sauge est un extrait aqueux, alcoolique, hydroalcoolique ou propylèneglycolique.

8. Composition selon la revendication 7, caractérisée par le fait que l'extrait de sauge est un extrait aqueux.

9. Composition selon la revendication 4, caractérisée par le fait que l'extrait mou de passiflore est un extrait aqueux ou hydroalcoolique.

10. Composition selon la revendication 9, caractérisée par le fait que l'extrait mou de passiflore a une teneur en solides comprise entre environ 60% et environ 80%.

**Revendications pour l'Etat contractant: AT**

1. Composition cosmétique caractérisée par le fait qu'elle contient dans un véhicule approprié pour une application cosmétique une association d'extraits végétaux constituée:

(i) d'un extrait de fragon (Ruscus aculeatus L), et

(ii) d'un extrait de sauge (Salvia officinalis L).

2. Composition selon la revendication 1, caractérisée par le fait qu'elle contient de 0,1 à 3% et de préférence de 0,3 à 2,5% en poids d'un extrait sec de fragon par rapport au poids total de la composition.

3. Composition selon la revendication 1, caractérisée par le fait qu'elle contient de 0,01 à 5% et de préférence de 0,3 à 3,5% en poids de l'extrait sec de sauge par rapport au poids total de la composition.

4. Composition selon l'une quelconque des revendications 1 à 3, caractérisée par le fait qu'elle contient en outre un extrait mou de passiflore (Passiflora incarnata) à une concentration (exprimée en matière solide) comprise entre 0,1 et 2% et de préférence entre 0,2 et 1,5% en poids par rapport au poids total de la composition.

5. Composition selon l'une quelconque des revendications 1 et 2, caractérisée par le fait que l'extrait de fragon est un extrait résultant de l'extraction à l'aide d'une solution hydroalcoolique d'un alcool ayant de 3 à 6 atomes de carbone et de préférence à l'aide de n-butanol saturé d'eau.

6. Composition selon la revendication 5, caractérisée par le fait que l'extrait sec de fragon a une teneur en saponines supérieure à 65% et de préférence comprise entre 70 et 80% en poids par rapport à l'extrait sec.

7. Composition selon l'une quelconque des revendications 1 et 3, caractérisée par le fait que l'extrait de sauge est un extrait aqueux, alcoolique, hydroalcoolique ou propylèneglycolique.

8. Composition selon la revendication 7, caractérisée par le fait que l'extrait de sauge est un extrait aqueux.

9. Composition selon la revendication 4, caractérisée par le fait que l'extrait mou de passiflore est un extrait aqueux ou hydroalcoolique.

10. Composition selon la revendication 9, caractérisée par le fait que l'extrait mou de passiflore a une teneur en solides comprise entre environ 60% et environ 80%.

**Patentansprüche für die Vertragsstaaten:**
**BE, CH, DE, FR, GB, IT, LI, NL**

1. Mittel zur kosmetischen oder pharmazeutischen Verwendung, das die Kapillarfragilität beeinflusst, dadurch gekennzeichnet, dass es in einem zur kosmetischen oder pharmazeutischen Anwendung geeigneten Träger eine Kombination aus Pflanzenextrakten enthält, welche aus

(i) einem Extrakt von Mäusedorn (Ruscus aculeatus L) und

(ii) einem Extrakt von Salbei (Salvia officinalis L) besteht.

2. Mittel nach Anspruch 1, dadurch gekennzeichnet, dass es 0,1 bis 3% und vorzugsweise 0,3 bis 2,5 Gew.-% eines Trockenextrakts von Mäusedorn enthält, bezogen auf das Gesamtgewicht des Mittels.

3. Mittel nach Anspruch 1, dadurch gekennzeichnet, dass es 0,01 bis 5% und vorzugsweise 0,3 bis 3,5 Gew.-% eines Trockenextrakts von Salbei enthält, bezogen auf das Gesamtgewicht des Mittels.

4. Mittel nach einem der Ansprüche 1 bis 3, dadurch gekennzeichnet, dass es ausserdem einen weichen Extrakt von der Passionsblume (Passiflora incarnata) in einer Konzentration (ausgedrückt als Feststoff) von 0,1 bis 2% und vorzugsweise von 0,2 bis 1,5 Gew.-% enthält, bezogen auf das Gesamtgewicht des Mittels.

5. Mittel nach einem der Ansprüche 1 und 2, dadurch gekennzeichnet, dass der Extrakt von Mäusedorn ein Extrakt ist, den man bei der Extraktion mit einer wässrige-alkoholischen Lösung eines Alkohols mit 3 bis 6 Kohlenstoffatomen und vorzugsweise mit n-Butanol, das mit Wasser gesättigt ist, erhält.

6. Mittel nach Anspruch 5, dadurch gekennzeichnet, dass der Trockenextrakt von Mäusedorn einen Gehalt an Saponinen grösser als 65% und vorzugsweise von 70 bis 80 Gew.-%, bezogen auf den Trockenextrakt, aufweist.

7. Mittel nach einem der Ansprüche 1 und 3, dadurch gekennzeichnet, dass der Extrakt von Salbei ein wässriger, alkoholischer, wässrig-alkoholischer oder propylenglykolischer Extrakt ist.

8. Mittel nach Anspruch 7, dadurch gekennzeichnet, dass der Extrakt von Salbei ein wässriger Extrakt ist.

9. Mittel nach Anspruch 4, dadurch gekennzeichnet, dass der weiche Extrakt von der Passionsblume ein wässriger oder wässrig-alkoholischer Extrakt ist.

10. Mittel nach Anspruch 9, dadurch gekennzeichnet, dass der weiche Extrakt von der Passionsblume einen Feststoffgehalt von etwa 60% bis etwa 80% besitzt.

**Patentansprüche für den Vertragsstaat: AT**

1. Kosmetisches Mittel, dadurch gekennzeichnet, dass es in einem zur kosmetischen Anwendung geeigneten Träger eine Kombination aus Pflanzenextrakten enthält, welche aus

(i) einem Extrakt von Mäusedorn (Ruscus aculeatus L) und

(ii) einem Extrakt von Salbei (Salvia officinalis L) besteht.

2. Mittel nach Anspruch 1, dadurch gekennzeichnet, dass es 0,1 bis 3% und vorzugsweise 0,3 bis 2,5 Gew.-% eines Trockenextrakts von Mäusedorn enthält, bezogen auf das Gesamtgewicht des Mittels.

3. Mittel nach Anspruch 1, dadurch gekennzeichnet, dass es 0,01 bis 5% und vorzugsweise 0,3 bis 3,5 Gew.-% eines Trockenextrakts von Salbei enthält, bezogen auf das Gesamtgewicht des Mittels.

4. Mittel nach einem der Ansprüche 1 bis 3, dadurch gekennzeichnet, dass es ausserdem einen weichen Extrakt von der Passionsblume (Passiflora incarnata) in einer Konzentration (ausgedrückt als Feststoff) von 0,1 bis 2% und vorzugsweise von 0,2 bis 1,5 Gew.-% enthält, bezogen auf das Gesamtgewicht des Mittels.

5. Mittel nach einem der Ansprüche 1 und 2, dadurch gekennzeichnet, dass der Extrakt von Mäusedorn ein Extrakt ist, den man bei der Extraktion mit einer wässrige-alkoholischen Lösung eines Alkohols mit 3 bis 6 Kohlenstoffatomen und vorzugsweise mit n-Butanol, das mit Wasser gesättigt ist, erhält.

6. Mittel nach Anspruch 5, dadurch gekennzeichnet, dass der Trockenextrakt von Mäusedorn einen Gehalt an Saponinen grösser als 65% und vorzugsweise von 70 bis 80 Gew.-%, bezogen auf den Trockenextrakt, aufweist.

7. Mittel nach einem der Ansprüche 1 und 3, dadurch gekennzeichnet, dass der Extrakt von Salbei ein wässriger, alkoholischer, wässrig-alkoholischer oder propylenglykolischer Extrakt ist.

8. Mittel nach Anspruch 7, dadurch gekennzeichnet, dass der Extrakt von Salbei ein wässriger Extrakt ist.

9. Mittel nach Anspruch 4, dadurch gekennzeichnet, dass der weiche Extrakt von der Passionsblume ein wässriger oder wässrig-alkoholischer Extrakt ist.

10. Mittel nach Anspruch 9, dadurch gekennzeichnet, dass der weiche Extrakt von der Passionsblume einen Feststoffgehalt von etwa 60% bis etwa 80% besitzt.

**Claims for the Contracting States:**
BE, CH, DE, FR, GB, IT, LI, NL

1. Composition for cosmetic or pharmaceutical use, which has an effect on the brittleness of hair, characterized in that it contains, in a vehicle suitable for a cosmetic or pharmaceutical application, a combination of plant extracts consisting of:
(i) an extract of butcher's broom (Ruscus aculeatus L), and
(ii) an extract of sage (Salvia officinalis L).

2. Composition according to Claim 1, characterized in that it contains from 0.1 to 3% and preferably from 0.3 to 2.5% by weight of a dry extract of butcher's broom relative to the weight of the composition.

3. Composition according to Claim 1, characterized in that it contains from 0.01 to 5% and preferably from 0.3 to 3.5% by weight of a dry extract of sage relative to the total weight of the composition.

4. Composition according to any one of Claims 1 to 3, characterized in that it additionally contains a soft extract of passiflora (Passiflora incarnata) at a concentration (expressed as dry matter) of between 0.1 and 2% and preferably between 0.2 and 1.5% by weight relative to the total weight of the composition.

5. Composition according to either of Claims 1 and 2, characterized in that the butcher's broom extract is an extract resulting from an extraction with an aqueous/alcoholic solution of an alcohol containing from 3 to 6 carbon atoms and preferably with water-saturated n-butanol.

6. Composition according to Claim 5, characterized in that the dry extract of butcher's broom has a saponin content greater than 65% and preferably between 70 and 80% by weight relative to the weight of the dry extract.

7. Composition according to either of Claims 1 and 3, characterized in that the extract of sage is an aqueous, alcoholic, aqueous/alcoholic or propylene glycolic extract.

8. Composition according to Claim 7, characterized in that the extract of sage is an aqueous extract.

9. Composition according to Claim 4, characterized in that the soft extract of passiflora is an aqueous or aqueous/alcoholic extract.

10. Composition according to Claim 9, characterized in that the soft extract of passiflora has a dry matter content of between approximately 60% and approximately 80%.

**Claims for the Contracting State: AT**

1. Cosmetic composition characterized in that it contains, in a vehicle suitable for a cosmetic application, a combination of plant extracts consisting of:
(i) an extract of butcher's broom (Ruscus aculeatus L), and
(ii) an extract of sage (Salvia officinalis L).

2. Composition according to Claim 1, characterized in that it contains from 0.1 to 3% and preferably from 0.3 to 2.5% by weight of a dry extract of butcher's broom relative to the weight of the composition.

3. Composition according to Claim 1, characterized in that it contains from 0.01 to 5% and preferably from 0.3 to 3.5% by weight of a dry extract of sage relative to the total weight of the composition.

4. Composition according to any one of Claims 1 to 3, characterized in that it additionally contains a soft extract of passiflora (Passiflora incarnata) at a concentration (expressed as dry matter) of between 0.1 and 2% and preferably between 0.2 and 1.5% by weight relative to the total weight of the composition.

5. Composition according to either of Claims 1 and 2, characterized in that the butcher's broom extract is an extract resulting from an extraction with an aqueous/alcoholic solution of an alcohol containing from 3 to 6 carbon atoms and preferably with water-saturated n-butanol.

6. Composition according to Claim 5, characterized in that the dry extract of butcher's broom has a saponin content greater than 65% and preferably between 70 and 80% by weight relative to the weight of the dry extract.

7. Composition according to either of Claims 1 and 3, characterized in that the extract of sage is an aqueous, alcoholic, aqueous/alcoholic or propylene glycolic extract.

8. Composition according to Claim 7, characterized in that the extract of sage is an aqueous extract.

9. Composition according to Claim 4, characterized in that the soft extract of passiflora is an aqueous or aqueous/alcoholic extract.

10. Composition according to Claim 9, characterized in that the soft extract of passiflora has a dry matter content of between approximately 60% and approximately 80%.